# EUROPEAN PATENT APPLICATION

(11) **EP 3 054 008 A1**
(43) Date of publication of application: **10.08.2016**
(21) Application number: 14850144.8
(22) Date of filing: 02.10.2014
(51) Int. Cl.: C12N 15/09

(54) **METHOD FOR PURIFYING DOUBLE-STRANDED RIBONUCLEIC ACID**

(30) Priority: 03.10.2013 JP 2013208548
(71) Applicant: Kyowa Hakko Bio Co., Ltd., Chiyoda-ku Tokyo 100-8185 (JP)
(72) Inventor: NAKANO, Tetsuo, Tokyo 100-8185 (JP); YAMAMURA, Eitora, Takaoka-shi Toyama 933-8511 (JP)
(74) Representative: Vossius & Partner Patentanwälte Rechtsanwälte mbB
(86) International application number: PCT/JP2014/076377
(87) International publication number: WO 2015/050191

(57) **Abstract**

The present invention provides a method of recovering dsRNA, from which a surfactant and endotoxin have been removed simultaneously, which includes mixing the surfactant and dsRNA, and contacting the mixture with a hydrophobic adsorptive resin or activated carbon.

## Description

### Technical Field

The present invention relates to a purification method of a double strand ribonucleic acid, comprising removing endotoxin from double strand ribonucleic acid.

### Background Art

Endotoxin derived from the outer membrane of a gram-negative bacterium is a highly active pyrogenic substance that causes high mortality diseases in living organisms such as human, animal and the like. Therefore, the endotoxin content of pharmaceutical products to be administered to human body and animals, such as injection preparation, injection vaccine and the like, needs to be suppressed to the lowest possible limit.

Endotoxin has extremely high physicochemical stability, and the physiological activity cannot disappear easily under mild conditions. For example, heating at 250°C for not less than 30 min is necessary for completely eliminating the physiological activity by a dry-heat treatment. While a method of eliminating biological activity by a treatment with an acid or base is also known (non-patent document 1, non-patent document 2, non-patent document 3), such radical treatment method cannot be used for removing endotoxin from biopolymer compounds such as protein preparation, nucleic acid preparation and the like.

As a method of removing endotoxin, a method using polyoxyethylene(8)octylphenylether is known (non-patent document 4). This method is a method of removing endotoxin from a protein solution based on a phase partition method. While the method can remove endotoxin under mild conditions of room temperature and neutrality, it has many problems such as the need to split solution into two phases by changing the temperature from room temperature to refrigerated temperature, indefinite boundary between the two phases, possible marked decrease in the recovery rate depending on the property of protein and the like. There is also a problem of polyoxyethylene(8)octylphenylether remaining at a high concentration in the protein solution after removal of endotoxin. In addition, it is unknown whether endotoxin contained in double strand ribonucleic acid (dsRNA) can be removed.

Biopolymers similar to dsRNA include deoxyribonucleic acid (DNA). Patent document 1 describes a method of removing endotoxin from plasmid DNA. This method is a method of removing host cell-derived impurities contaminating the plasmid DNA by selectively binding the impurities to a hydrophobic adsorptive substance. The host cell impurities defined in patent document 1 also includes RNA besides endotoxin, and is not a method for removing endotoxin from RNA. In this method, moreover, since all RNAs including RNA that formed a double strand such as transfer RNA, ribosomal RNA and the like, as well as messenger RNA bind to a hydrophobic interactive substance together with endotoxin, RNA cannot be purified. In addition, since a sufficient salt needs to be added in advance, a subsequent step for removing the salt should be added.

There is no known method for removing endotoxin from dsRNA applicable to industrial large-scale production process. While there are many known methods for removing endotoxin from protein and DNA, they have problems such as temperature, pH, salt, amount of residual surfactant, adsorption to hydrophobic interactive medium and the like for application as a method for removing endotoxin from dsRNA.

To use dsRNA for pharmaceutical products or use similar thereto, a method of effectively removing endotoxin needs to be established. The present invention provides a means for removing endotoxin from dsRNA that can be applied to industrial large-scale production process.

### [Document List]

### Patent Document

patent document 1: JP-A-2012-50463

### Non-patent Documents

non-patent document 1: Journal of Bacteriology, 1969, vol. 97, p. 1069-1077
non-patent document 2: Ann. N. Y. Acad. Sci., 1966, vol. 133, P. 604-621
non-patent document 3: Biochem. Biophys. Res. Commun., 1981, vol. 101, P. 434-439
non-patent document 4: Journal of Immunological Methods, 1990, vol. 132, p. 191-195

### SUMMARY OF THE INVENTION

### Problems to be Solved by the Invention

An object of the present invention is to provide a purification method of dsRNA or a salt thereof, which removes endotoxin almost completely.

### Means of Solving the Problems

The present inventors have conducted intensive studies in an attempt to solve the aforementioned problem and found that endotoxin and a surfactant can be removed and purified from a dsRNA aqueous solution by mixing the surfactant with the dsRNA aqueous solution containing endotoxin and contacting them with a hydrophobic adsorptive resin or activated carbon.

The present inventors have conducted further studies based on the above-mentioned findings and completed the present invention.

That is, the present invention provides
[1] A purification method of double strand ribonucleic acid (dsRNA) comprising mixing a surfactant with an aqueous dsRNA solution containing endotoxin, standing the mixture, and contacting the mixture with a hydrophobic adsorptive resin or activated carbon to remove said endotoxin and said surfactant;
[2] the purification method of [1], wherein the surfactant is a nonionic surfactant, an amphoteric surfactant, or an anionic surfactant;
[3] the purification method of [2], wherein the nonionic surfactant is a polyoxyethylene alkyl ether nonionic surfactant;
[4] the purification method of [2], wherein the amphoteric surfactant is a sulfobetaine amphoteric surfactant;
[5] the purification method of [2], wherein the anionic surfactant is a steroid anionic surfactant;
[6] the purification method of any one of the above-mentioned [1] - [5], wherein the hydrophobic adsorptive resin is a hydrophobic adsorptive resin selected from the group consisting of styrenic hydrophobic adsorptive resin, acrylic hydrophobic adsorptive resin, methacrylic hydrophobic adsorptive resin, and surfactant removal resin.

### Effect of the Invention

Safe dsRNA or a salt thereof from which endotoxin has been removed almost completely can be produced and provided at an industrial scale.

### Description of Embodiments

dsRNA to which the purification method of the present invention can be applied may be any as long as it has a structure wherein ribonucleic acid forms a double strand. For example, it may be dsRNA consisting of a single strand ribonucleic acid (ssRNA) and a single strand ssRNA complementary to the ssRNA, or RNA having a stem-loop structure formed by intramolecular annealing between complementary sequences in a single ssRNA. Furthermore, the dsRNA of the present invention may be in a salt form, and metal salt, ammonium salt, organic amine addition salt, amino acid addition salt and the like can be mentioned. Examples of the metal salt include alkali metal salts such as sodium salt, potassium salt and the like, alkaline earth metal salts such as magnesium salt, calcium salt and the like, aluminum salt, zinc salt and the like. Examples of the ammonium salt include salts such as ammonium, tetramethylammonium and the like. Examples of the organic amine addition salt include salts such as trishydroxyaminomethane and the like. Examples of the amino acid addition salt include salts such as lysine, arginine, histidine, tryptophan, ornithine and the like.

The kind, sequence and base number of the base of ssRNA constituting dsRNA are not limited as long as it has complementarity of a level capable of forming a double strand between molecules of ssRNA or in the molecules of ssRNA. As used herein, "having complementarity of a level capable of forming a double strand" means that it has complementarity of not less than about 60%, preferably not less than about 70%, more preferably not less than about 80%, further preferably not less than about 90%, further more preferably not less than about 95%, most preferably not less than about 98%, between base sequences between ssRNA molecules or in ssRNA molecule. Complementarity between the bases of ribonucleic acid is well known in the technical field. As the base constituting each ssRNA, for example, adenine, uracil, guanine, cytosine, or structural analogs thereof and the like can be mentioned, and each ssRNA may be constituted of the same base or bases different from each other.

While the number of base pairs of dsRNA is not questioned, it is desirably 10 - 10,000 base pairs, more desirably 10 - 2,000 base pairs.

The dsRNA aqueous solution may or may not contain a salt. The salt concentration may be any and not more than 150 mM is a practical and preferable range. The kind of the salt include acetate, carbonate, phosphate, ammonium salt, SO₄²⁻, Cl⁻, Br⁻, NO₃⁻, Mg²⁺, Li⁺, Na⁺, K⁺ and NH₄⁺, and also includes a mixture of anion or cation selected from the group not limited thereto. It may also contain a substance having a buffering action besides salts. Examples of the substance having a buffering action include Tris, TES, phosphate, Tricine, PIPES, HEPES, MOPS, MEPES, MES, Bicine and the like.

The surfactant in the present invention to be mixed with the aforementioned dsRNA aqueous solution is not particularly limited as long as it is an amphipathic molecule having a hydrophobic group and a hydrophilic group and, for example, nonionic surfactant, amphoteric surfactant, or anionic surfactant can be mentioned.

Examples of the nonionic surfactant include polyoxyethylene alkyl ethers, alkylpolyglucosides, fatty acid diethanolamides, alkylmonoglycerylethers, and fatty acid sorbitan esters, preferably polyoxyethylene alkyl ethers, most preferably polyoxyethyleneoctylphenylether.

Examples of the amphoteric surfactant include alkyldimethylamineoxides, alkylcarboxybetaines, and sulfobetaines, preferably sulfobetaines, most preferably 3-[(3-cholamidepropyl)dimethylammonio]-1-propanesulfonate.

Examples of the anionic surfactant include alkylbenzenesulfonates, monoalkylphosphates, alkylpolyoxyethylenesulfates, monoalkylsulfates, fatty acid sodiums, and steroids, preferably steroids, particularly preferably deoxycholic acid salt, most preferably deoxysodium cholate.

The surfactant may or may not be dissolved, and the final concentration (V/V) on mixing is 0.001% - 70%, preferably 0.01% - 50%, more preferably 0.1% - 10%. While the temperature during mixing is not limited, stable removing performance can be obtained by keeping the temperature constant, and the temperature is generally lower than 100°C under normal pressure, preferably not more than 40°C, more preferably not more than 15°C.

The aforementioned dsRNA aqueous solution and a surfactant are mixed and stood. The temperature during standing may be a temperature at which dsRNA is stable. Generally, it is a temperature lower than 100°C under normal pressure, preferably not more than 40°C, more preferably not more than 15°C. The time of standing is 1 sec - 10 days, preferably 1 min - 1 day, more preferably 10 min to 5 hr.

The hydrophobic adsorptive resin or activated carbon in the present invention to be contacted with the aforementioned mixture is not particularly limited as long as it selectively adsorbs a surfactant and endotoxin when a solution containing dsRNA, the surfactant and endotoxin is contacted with the resin or activated carbon.

Examples of the above-mentioned hydrophobic adsorptive resin (to be also referred to as synthetic adsorptive resin) include styrenic hydrophobic adsorptive resin, acrylic hydrophobic adsorptive resin, and methacrylic hydrophobic adsorptive resin. As these preferable resins, examples of the styrenic hydrophobic adsorptive resin include sepabeads (registered trade mark) SP207, SP70 (Mitsubishi Chemical Co., Ltd., Japan), duolite (registered trade mark) S874, S876, S877 (Sumika Chemtex Co. Ltd), and amberlite (registered trade mark) XAD2000, XAD4, FPX66, XAD1180N, XAD-2 (Organo Co. Ltd., Japan) and the like, examples of the acrylic hydrophobic adsorptive resin include amberlite (registered trade mark) XAD7HP (Organo Co. Ltd., Japan), and examples of the methacrylic hydrophobic adsorptive resin include diaion (registered trade mark) HP2MG (Mitsubishi Chemical Co., Ltd., Japan) and the like.

Also, a resin mixture wherein plural hydrophobic adsorptive resins and silica beads are three dimensionally cross-linked, which is sold as a resin for removing surfactant, is also included in the hydrophobic adsorptive resin. The hydrophobic adsorptive resin to be three dimensionally cross-linked with the above-mentioned silica beads is not particularly limited as long as it selectively adsorbs a surfactant and endotoxin. Examples thereof include styrenic hydrophobic adsorptive resin, acrylic hydrophobic adsorptive resin, and methacrylic hydrophobic adsorptive resin. In addition, commercially available resins for removing surfactants can also be used preferably in the present invention. Examples of such resin for removing surfactant include CALBIOSORB (EMD Millipore, USA) or SDR HyperD (registered trade mark) (Pall Corp., USA), preferably SDR HyperD (registered trade mark).

The above-mentioned activated carbon is not particularly limited as long as it maintains the property to adsorb surfactants by an intermolecular interaction force, and can selectively adsorb a surfactant and endotoxin when a solution containing dsRNA, the surfactant and endotoxin is contacted with activated carbon. As such activated carbon, commercially available activated carbon can be used and, for example, granular activated carbon SHIRASAGI (registered trade mark) LH2C can be mentioned.

The shape of the hydrophobic adsorptive resin or activated carbon to be used in the present invention may be any, and can be granular, membrane-like, disrupted, fiber-like and the like. When the resin contains a bead-like carrier such as silica gel and the like, the size of the bead carrier is not limited. The above-mentioned hydrophobic adsorptive resin is not limited as to the presence or absence of pore, number of pore, and pore size.

The aforementioned mixture after standing can be contacted with a hydrophobic adsorptive resin or activated carbon by those of ordinary skill in the art according to a known method.

One of such method is, for example, a column chromatography method. In the column chromatography method, the aforementioned hydrophobic adsorptive resin or activated carbon is packed in a suitable liquid chromatography column, equilibrated, and the aforementioned mixture after standing is applied, whereby a dsRNA aqueous solution free of endotoxin and surfactant can be recovered. While a solvent to be used for equilibration of the hydrophobic adsorptive resin or activated carbon or recovery of the dsRNA aqueous solution is not particularly limited as long as it is a highly polar solvent, for example, water or water containing a salt (e.g., ammonium sulfate, sodium citrate, potassium citrate, potassium phosphate, sodium phosphate, sodium sulfate, potassium sulfate, magnesium sulfate, sodium chloride, potassium chloride etc.) is used. While the temperature during contact is not limited, stable performance can be obtained by keeping the temperature constant, and the temperature is preferably 0 - 40°C, more preferably 10 - 30°C.

Another method is a batch method. In a batch method, the aforementioned hydrophobic adsorptive resin or activated carbon is filled in a suitable container and equilibrated, the aforementioned mixture after standing is added, and the mixture is stood, centrifuged or filtered and the like, whereby a dsRNA aqueous solution free of endotoxin and surfactant can be recovered. In addition, the solvent, temperature and the like to be used for equilibration of a hydrophobic adsorptive resin or activated carbon can be the same as those in the column chromatography method.

### Examples

The present invention is explained in more detail in the following by referring to Examples, which are not to be construed as limitative.

### Example 1. Removal of endotoxin by using polyoxyethylene(8)octylphenylether and resin for removing surfactant

A mixture dsRNA1-M composed of 0.5 ml of a double strand ribonucleic acid solution dsRNA1-C (Abs absorbance at 260 nm = 200) having an average chain length of about 320 base pairs, which is composed of a polyinosinic acid polymer and a polycytidylic acid polymer and from which contaminating salt was removed by UF membrane dialysis, 0.02 ml of ether type nonionic surfactant polyoxyethylene(8)octylphenylether (manufactured by Wako Pure Chemical Industries, Ltd.), and 0.48 ml of ultrapure water was produced, and stood at 4°C for 2 hr. 1 ml of a resin SDR HyperD^{R} for removing surfactant (manufactured by Pall corporation) was packed in Poly-Prep^{R} Chromatography Columns (manufactured by Bio-Rad Laboratories, Inc) to give a resin column, and the resin was equilibrated by applying 5 ml of ultrapure water. To the resin column was applied 0.25 ml of dsRNA1-M, ultrapure water was sequentially added dropwise, and 0.25 ml each of the eluate was sampled. Two fractions showing high absorbance intensity at Abs 260 nm were combined to give 0.5 ml of dsRNA1-P. The absorbance intensity of dsRNA1-P at Abs 260 nm was 36.

### Example 2. Measurement of endotoxin concentration

The endotoxin concentration of dsRNA1-C was analyzed using a commercially available endotoxin quantitative analysis kit Toxin Sensor™ Endotoxin Detection System (manufactured by GenScript USA Inc.). As a result of the measurement according to the instruction manual, the endotoxin concentration of dsRNA1-C was 3.0 EU/ml and, by conversion to endotoxin amount per absorbance intensity at Abs 260 nm, 0.030 EU/Abs (260 nm). Similarly, the endotoxin concentration of dsRNA1-P was measured; however, endotoxin could not be detected. Since the marginal by this measurement method is 0.005 EU/ml, when converted to the endotoxin amount per absorbance intensity at Abs 260 nm, it was less than 0.0000139 EU/Abs (260 nm).

### Example 3. Measurement of polyoxyethylene(8)octylphenylether concentration

A quantitative analysis of polyoxyethylene(8)octylphenylether was performed by high performance liquid chromatography (HPLC). Samples were diluted with 30 % methanol and subjected to the HPLC analysis. The analysis conditions were as follows.
- eluent A: ultrapure water
- eluent B: 100 % methanol
- gradient: gradient elution with 60 - 80 % methanol
- pump flow rate: 0.5 ml/min
- column: Inertsil ODS-3, column length 150 mm x 3.0 mm, particle size 3 µm (GL Sciences Inc.)
- column temperature: 50°C
- detector: Abs 223 nm

Using polyoxyethylene(8)octylphenylether, an analytical curve was drawn to find the marginal of 0.5 v/v ppm. Polyoxyethylene(8)octylphenylether contained in dsRNA1-P was measured by this method to find that the level was below marginal.

### Example 4. Removal of endotoxin by batch method using resin for removing surfactant

A mixture dsRNA2-M composed of 1 ml of a double strand ribonucleic acid solution dsRNA2-C (Abs absorbance at 260 nm = 200) having an average chain length of about 280 base pairs, which is composed of a polyinosinic acid polymer and a polycytidylic acid polymer and from which contaminating salt was removed by UF membrane dialysis, 0.04 ml of 99% polyoxyethylene(8)octylphenylether (manufactured by Wako Pure Chemical Industries, Ltd.), and 0.96 ml of ultrapure water was produced, and stood at 4°C for 2 hr.

0.5 ml of SDR HyperD^{R} was packed in a 2.0 ml plastic sampling tube. Then, ultrapure water (1 ml) was added and the mixture was centrifuged at 8000xg, 15 sec and the supernatant was removed by a washing operation. The washing operation was performed 5 times in total to equilibrate SDR HyperD^{R}. dsRNA2-M (0.5 ml) was added, and the mixture was reciprocally shaken at room temperature for 1 hr. After shaking, the mixture was stood and the resulting supernatant dsRNA2-P-SD was collected.

### Example 5. Removal of endotoxin by batch method using hydrophobic adsorptive resin

0.5 ml of hydrophobic adsorptive resin sepabeads (registered trade mark) SP207 (manufactured by Mitsubishi Chemical Co., Ltd.) was packed in a 2.0 ml plastic sampling tube. Then, ultrapure water (1 ml) was added and the mixture was centrifuged at 8000xg, 15 sec and the supernatant was removed by 5 times in total of a washing operation to equilibrate SP207. dsRNA2-M (0.5 ml) produced in Example 4 was added, and the mixture was reciprocally shaken at room temperature for 1 hr. After shaking, the mixture was stood and the resulting supernatant dsRNA2-P-SP was collected.

### Example 6. Removal of endotoxin by batch method using activated carbon

0.5 ml of granular activated carbon SHIRASAGI (registered trade mark) LH2C (manufactured by Japan EnviroChemicals) was packed in a 2.0 ml plastic sampling tube. Then, ultrapure water (1 ml) was added and the mixture was centrifuged at 8000xg, 15 sec and the supernatant was removed by 5 times in total of a washing operation to equilibrate SHIRASAGI (registered trade mark) LH2C. dsRNA2-M (0.5 ml) produced in Example 4 was added, and the mixture was reciprocally shaken at room temperature for 1 hr. After shaking, the mixture was stood and the resulting supernatant dsRNA2-P-LH was collected.

### Example 7. Measurement of endotoxin concentration

The concentration of polyoxyethylene(8)octylphenylether contained in dsRNA2-P-SD and dsRNA2-P-SP and dsRNA2-P-LH was measured by the method of Example 3 to find that the level was below marginal for each.

The results of absorbance intensity and endotoxin concentration of dsRNA2-C and dsRNA2-P-SD, dsRNA2-P-SP, dsRNA2-P-LH as measured by the method of Example 3 are summarized in Table 1.

When treated with sepabeads (registered trade mark) SP207 or granular activated carbon SHIRASAGI (registered trade mark) LH2C, endotoxin was reduced to the same level as or lower level than that by SDR HyperD^{R}, and the high yield of dsRNA was almost equivalent.

**Table 1**

| sample | absorbance intensity (Abs 260 nm) | endotoxin concentration (EU/ml) | endotoxin concentration per absorbance intensity (EU/Abs 260 nm) |
|---|---|---|---|
| dsRNA2-C | 200 | 61.65 | 0.3083 |
| dsRNA2-P-SD | 72 | 0.18 | 0.0025 |
| dsRNA2-P-SP | 81 | 0.20 | 0.0025 |
| dsRNA2-P-LH | 68 | 0.10 | 0.0015 |

### Example 8. Removal of endotoxin by using polyoxyethylene(10)octylphenylether

The clouding point of polyoxyethylene(8)octylphenylether is 25°C when a salt is not contained. To examine the relationship between the clouding point and the endotoxin removal actions shown in Example 1 and Example 4, a test using polyoxyethylene(10)octylphenylether having a lower clouding point than polyoxyethylene(8)octylphenylether was performed.

A mixture of 0.5 ml of an aqueous dsRNA2-C solution (Abs absorbance at 260 nm = 200), 0.02 ml of 99% polyoxyethylene(10)octylphenylether (manufactured by Wako Pure Chemical Industries, Ltd.), and 0.48 ml of ultrapure water was produced, and stood at 4°C for 2 hr. 0.5 ml was added to a 2.0 ml plastic sampling tube packed with 0.5 ml of SDR HyperD^{R} equilibrated in the same manner as in Example 4 and the mixture was reciprocally shaken at room temperature for 1 hr. After shaking, the mixture was stood and the resulting supernatant dsRNA2-P-SD-10 was collected. The endotoxin concentration of dsRNA2-P-SD-10 was analyzed by the procedure shown in Example 2. The results are summarized in Table 2 together with the values of dsRNA2-C and dsRNA2-P-SD shown in Example 4.

In addition, a mixture of 0.5 ml of an aqueous dsRNA2-C solution (Abs absorbance at 260 nm = 200) was diluted two-fold with 0.5 ml of ultrapure water, and the solution (0.5 ml) was added to a 2.0 ml plastic sampling tube packed with 0.5 ml of SDR HyperD^{R} equilibrated in the same manner as in Example 4 and the mixture was reciprocally shaken at room temperature for 1 hr. After shaking, the mixture was stood and the resulting supernatant dsRNA2-P-SD-N was collected and used as a control example. The endotoxin concentration of dsRNA2-P-SD-N was analyzed by the procedure shown in Example 2.

**Table 2**

| sample | absorbance intensity (Abs 260 nm) | endotoxin concentration (EU/ml) | endotoxin concentration per absorbance intensity (EU/Abs 260 nm) |
|---|---|---|---|
| dsRNA2-C | 200 | 61.65 | 0.3083 |
| dsRNA2-P-SD-N | 68 | 5.88 | 0.0865 |
| dsRNA2-P-SD | 72 | 0.18 | 0.0025 |
| dsRNA2-P-SD-10 | 81 | 0.20 | 0.0025 |

As is clear from Table 2, the endotoxin concentration of dsRNA2-P-SD-N which was contacted with the resin without addition of polyoxyethyleneoctylphenylether was high. However, the endotoxin concentration of dsRNA2-P-SD-10 added with polyoxyethylene(10)octylphenylether was equivalent to that of dsRNA2-P-SD added with polyoxyethylene(8)octylphenylether. Therefrom it was clarified that the endotoxin removal action in the present invention does not depend on the clouding point of the surfactant.

### Example 9. Removal of endotoxin by using 3-[(3-cholamidepropyl)dimethylammoniol-1-propanesulfonate

A mixture of 0.5 ml of an aqueous dsRNA2-C solution (Abs absorbance at 260 nm = 200), 0.02 ml of 3-[(3-cholamidepropyl)dimethylammonio]-1-propanesulfonate (manufactured by DOJINDO LABORATORIES), and 0.48 ml of ultrapure water was produced, and stood at 4°C for 2 hr. The solution (0.5 ml) was added to a 2.0 ml plastic sampling tube packed with 0.5 ml of SDR HyperD^{R} equilibrated in the same manner as in Example 4 and the mixture was reciprocally shaken at room temperature for 1 hr. After shaking, the mixture was stood and the resulting supernatant dsRNA2-P-SD-CH was collected. In the same manner as in Example 2, the endotoxin concentration of dsRNA2-SD-CH and absorbance intensity were measured. As a result, superior removal of endotoxin was observed as evidenced by absorbance intensity 74, endotoxin concentration 0.18 EU/ml, and endotoxin concentration per absorbance intensity 0.0025 EU/Abs (260 nm).

### Example 10. Removal of endotoxin by using deoxycholic acid

A mixture of 0.5 ml of an aqueous dsRNA2-C solution (Abs absorbance at 260 nm = 200) and 0.5 ml of 4%(w/v) deoxysodium cholate (manufactured by Wako Pure Chemical Industries, Ltd.) was produced, and stood at 4°C for 2 hr. The solution (0.5 ml) was added to a 2.0 ml plastic sampling tube packed with 0.5 ml of SDR HyperD^{R} equilibrated in the same manner as in Example 4 and the mixture was reciprocally shaken at room temperature for 1 hr. After shaking, the mixture was stood and the resulting supernatant dsRNA2-P-SD-DE was collected.

In the same manner as in Example 2, the endotoxin concentration of dsRNA2-SD-DE and absorbance intensity were measured. As a result, superior removal of endotoxin was observed as evidenced by absorbance intensity 74, endotoxin concentration 0.18 EU/ml, and endotoxin concentration per absorbance intensity 0.0025 EU/Abs (260 nm).

### Example 11. Influence of salt concentration

A mixture of 0.5 ml of an aqueous dsRNA2-C solution (Abs absorbance at 260 nm = 200), 0.25 ml of 0.6 M NaCl solution, 0.23 ml of ultrapure water and 0.02 ml of polyoxyethylene(8)octylphenylether was produced, and stood at 4°C for 2 hr. The solution was added to a 2.0 ml plastic sampling tube packed with 0.5 ml of SDR HyperD^{R} equilibrated in the same manner as in Example 4 and the mixture was reciprocally shaken at room temperature for 1 hr. After shaking, the mixture was stood and the resulting supernatant (dsRNA2-P-SD-NA) was collected. The endotoxin concentration of dsRNA2-P-SD-NA was quantitatively analyzed, and compared with dsRNA2-P-SD of Example 4. The results are summarized in Table 3. As is clear from Table 3, endotoxin was removed without relying on the NaCl concentration of the samples.

**Table 3**

| sample | absorbance intensity (Abs 260 nm) | endotoxin concentration (EU/ml) | endotoxin concentration per absorbance intensity (EU/Abs 260 nm) |
|---|---|---|---|
| dsRNA2-C | 200 | 61.65 | 0.3083 |
| dsRNA2-P-SD | 72 | 0.18 | 0.0025 |
| dsRNA2-P-SD-NA | 72 | 0.18 | 0.0025 |

### Industrial Applicability

Safe dsRNA or a salt thereof, from which endotoxin has been removed almost completely, can be produced and provided at an industrial scale, and can be used with ease as a pharmaceutical product or for use analogous thereto.

This application is based on a patent application No. 2013-208548 filed in Japan (filing date: October 3, 2013), the contents of which are incorporated in full herein.

## Claims

1. A purification method of double strand ribonucleic acid (dsRNA) comprising mixing a surfactant with an aqueous dsRNA solution containing endotoxin, standing the mixture, and contacting the mixture with a hydrophobic adsorptive resin or activated carbon to remove said endotoxin and said surfactant.

2. The purification method according to claim 1, wherein the surfactant is a nonionic surfactant, an amphoteric surfactant, or an anionic surfactant.

3. The purification method according to claim 2, wherein the nonionic surfactant is a polyoxyethylene alkyl ether nonionic surfactant.

4. The purification method according to claim 2, wherein the, amphoteric surfactant is a sulfobetaine amphoteric surfactant.

5. The purification method according to claim 2, wherein the anionic surfactant is a steroid anionic surfactant.

6. The purification method according to any one of claims 1 to 5, wherein the hydrophobic adsorptive resin is a hydrophobic adsorptive resin selected from the group consisting of styrenic hydrophobic adsorptive resin, acrylic hydrophobic adsorptive re sin, methacrylic hydrophobic adsorptive resin, and surfactant removal resin.
